# EUROPEAN PATENT APPLICATION

(11) **EP 3 569 225 A1**
(43) Date of publication of application: **20.11.2019**
(21) Application number: 18173268.6
(22) Date of filing: 18.05.2018
(51) Int. Cl.: A61K 9/20, A61K 31/427

(54) **SOLID DISPERSION CONTAINING RITONAVIR**

(71) Applicant: Pharmaceutical Oriented Services Ltd, 14452 Metamorfossi Attikis (GR); Remedica Ltd, 3056 Limassol (CY)
(72) Inventor: Panagopoulos, Panagiotis, 14452 Metamorfossi Attikis (GR); Panitsas, Konstantinos-Emmanouil, 14452 Metamorfossi Attikis (GR); Komporozos, Konstantinos, 14452 Metamorfossi Attikis (GR); Likoudis, Aggelos, 14452 Metamorfossi Attikis (GR); Syrigos, Matthaios, 14452 Metamorfossi Attikis (GR); Hatzouli, Maria, 14452 Metamorfossi Attikis (GR); Pattihis, Charalambos, 3508 Limassol (CY); Nordmann, Antje, 3508 Limassol (CY); Michalis, Neoptolemou, 3508 Limassol (CY)
(74) Representative: Hamm&Wittkopp Patentanwälte PartmbB

(57) **Abstract**

The present invention relates to a solid dispersion containing ritonavir and a cationic polymer, and to the use of the solid dispersion for the preparation of a pharmaceutical composition for oral administration, which is preferably a tablet. The pharmaceutical composition is intended for the treatment of HIV and may additionally contain a HIV protease inhibitor selected from darunavir, lopinavir, atazanavir, tipranavir and saquinavir.

## Description

The present invention relates to a solid dispersion containing ritonavir and to its use in the preparation of a solid pharmaceutical composition.

Ritonavir has a low solubility in aqueous media as well as low membrane permeability, so that this drug is classified as a class IV compound according to the Biopharmaceutics Classification System (BCS). Class IV drugs are generally difficult to be formulated as a unit dosage form for oral administration, because adequate oral bioavailability cannot easily be achieved.

Various techniques have been employed in the state of the art in order to enhance the oral bioavailability of BCS class IV drugs, e.g. lipid based delivery systems, polymer based nano-carriers, crystal engineering (nano-crystals and co-crystals), liquisolid technology and self-emulsifying solid dispersions (Journal of Controlled Release 2017, 248, 71-95). Self-emulsifying solid dispersions consist of a dispersion of the drug in an inert excipient matrix, where the drug may exist in finely divided crystalline or amorphous states or in a molecularly dispersed form (solid solution).

Typical self-emulsifying polymers, which may be employed as pharmaceutical excipients constituting the matrix of the solid dispersion, are lauroyl polyoxylglycerides (e.g. PEG-32 lauroyl polyoxylglyceride (Gelucire® 44/14)), tocopheryl polyethylene glycol 1000 succinate (TPGS) or polyoxyethylene/polyoxypropylene copolymer (poloxamer (Pluronics®)).

Ritonavir is marketed under the tradename Norvir® in the form of a soft capsule or tablet. In addition, a fixed combination of ritonavir and lopinavir is marketed under the tradename Kaletra® in the form of a soft capsule or tablet. The mono and the combination product are indicated in combination with other antiretroviral agents for the treatment of HIV-1 infection.

Two polymorphs of ritonavir (forms I and II) are known. The preparation of these forms as well as the preparation of amorphous ritonavir is described in WO 00/04016. Form II is the thermodynamically stable form and is much less soluble than form I. Hence, there is a high risk that a unit dosage form containing ritonavir form I may exhibit a dramatic decrease of oral bioavailability if the form I transforms into form II during storage. The Norvir® and Kaletra® soft capsules were developed in order to suppress the formation of the form II. The Norvir® soft capsule contains a solution of ritonavir in ethanol, oleic acid, water and polyoxyl 35 castor oil. In the Kaletra^{®} soft capsule, propylene glycol is used instead of ethanol. The crystallization of form II ritonavir is suppressed if the capsules are stored at 5 °C.

The Norvir® and Kaletra^{®} film-coated tablets show, compared to the soft capsule formulations, both improved storage stability (the tablets may be stored at room temperature without formation of form II ritonavir) and enhanced oral bioavailability. The tablet cores contain copovidone, sorbitan monolaurate, colloidal anhydrous silica and sodium stearyl fumarate, whereby the Norvir® tablet additionally contains anhydrous calcium hydrogen phosphate. The tablets are prepared by hot-melt extrusion, which affords a solid solution of the active substances in the matrix. The main excipient is copovidone, a copolymer of vinylpyrrolidone and vinyl acetate (60 %/40 %).

WO 01/34118 describes a solid dispersion containing ritonavir. The matrix is formed by a water-soluble carrier, e.g, by polyethylene glycol (PEG), poloxamer, polyoxyethylene stearate or poly-ε-caprolactone. The solid dispersion is prepared by dissolving ritonavir in an organic solvent, preferably ethanol, dissolving the drug in the water-soluble polymer by adding the polymer to the solution and removing the organic solvent by evaporation. The preferred water-soluble polymer is PEG. The dissolution rate of ritonavir depends on the drug load: the higher the drug load, the lower the drug release from particles containing ritonavir dissolved in PEG. The *in vitro* dissolution profile correlates with the oral bioavailability; the best oral bioavailability was found with the lowest drug load.

WO 2005/039551 and WO 2006/091529 relate to the Kaletra^{®} tablet. They describe a solid solution containing ritonavir and lopinavir within a water-soluble polymer, whereby the solid solution is prepared by hot-melt extrusion. The preferred water-soluble polymer is copovidone. In order to increase the oral bioavailability of the drugs, the blend to be subjected to melt-extrusion should contain a surfactant having a hydrophilic-lipophilic balance (HLB) value of 4-10. The preferred surfactants are sorbitan monolaurate and sorbitan monopalmitate.

WO 2009/081174 discloses a bilayer tablet containing darunavir in one layer and ritonavir in the other layer. It is stated in the application that the drugs may be dissolved/dispersed by hot-melt extrusion within a matrix containing a water-soluble and/or water-insoluble polymer, whereby the drug : polymer ratio should range from 1 : 1 to 1 : 6. The preferred water-soluble polymer is copovidone, while examples of water-insoluble polymers include acrylic copolymers (e.g. Eudragit® E), polyvinyl acetate, ethyl cellulose and cellulose acetate. The darunavir-layer is preferably prepared by wet-granulation in which a mixture of darunavir, microcrystalline cellulose and crospovidone is treated with a polyvinylpyrrolidone-containing aqueous solution. The ritonavir-layer is preferably prepared by holt-melt extrusion of a mixture containing the drug, copovidone and sorbitan monolaurate or polyoxyethylene castor oil (Cremophor®).

The ritonavir-containing tablets described in the prior art solve the oral bioavailability problem of the BCS class IV drug ritonavir by formulating the drug as a solid dispersion, i.e. by dissolving the drug in a self-emulsifying water-soluble polymer matrix. Instead of using self-emulsifying polymers, as Gelucire® 44/14, TPGS or poloxamers, a combination of a water-soluble polymer and a surfactant is employed. It was an objective of the present invention to provide an alternative ritonavir-containing pharmaceutical composition, in which the formation of crystalline ritonavir, in particular of form II ritonavir is suppressed and which shows adequate oral bioavailability. This objective is attained by the subject matter as defined in the claims.

The present invention relates to a solid dispersion comprising ritonavir, a cationic polymer containing an amino group, and optionally a pharmaceutical excipient. It is preferred that the solid dispersion contains ritonavir in a molecularly dispersed form (solid solution). In another embodiment of the present invention, the solid dispersion may contain particles of ritonavir in the amorphous state.

The solid dispersion of the present invention serves the purpose of enhancing oral bioavailability of ritonavir. After oral administration of the solid dispersion, the drug dissolves along with the cationic polymer containing an amino group to create a supersaturated solution. After dissolution of a solid dispersion in the gastrointestinal fluid, the drug may crystallize, so that the dissolution advantage of the solid dispersion is lost. In the prior art formulations, the crystallization of ritonavir was suppressed by suspending the drug in a matrix of a water-soluble polymer and a surfactant. According to the present invention, no surfactant is required if ritonavir is dispersed in a cationic polymer containing an amino group.

It was found that a cationic polymer containing an amino group suppresses the crystallization of ritonavir in the gastrointestinal fluid. Cationic polymers with amino groups have water solubility at acidic pH but not at neutral pH or above pH 7. This is achieved by the presence of the amino group that is protonated under acidic conditions. The cationic polymer used in the present invention is soluble in water at a pH of 6.5 or below, preferably at a pH of 6 or below. It is assumed that the water penetration into the solid dispersion is delayed until the solid dispersion reaches the stomach. In the stomach (pH 1-3.5) the solid dispersion dissolves due to the protonation of the cationic polymer.

The weight ratio of the cationic polymer to ritonavir is usually 6:1 to 1:2, preferably 3 : 1 to 2 : 3, more preferred 3 : 2 to 1 : 1.

Preferably, the cationic polymer is selected from aminoalkyl methacrylate copolymer, polyvinylacetal diethylaminoacetate and chitosan. As an example of the aminoalkyl methacrylate copolymer butyl methacrylate/(2-dimethylaminoethyl)-methacrylate/methyl methacrylate copolymer (1 : 2 : 1), which is marketed under the tradename Eudragit® E, may be used. Chitosan is soluble in water at a pH of 6.5 and below; Eudragit® E is soluble in water at a pH of 5 or below, while polyvinylacetal diethylaminoacetate is soluble in water at a pH of 5.8 and below.

The solid dispersion of the present invention may contain a pharmaceutical excipient selected from a filler, a disintegrant and a surfactant.

Examples of fillers include microcrystalline cellulose, calcium hydrogen phosphate (anhydrous or dihydrate), lactose (anhydrous or monohydrate), calcium carbonate, magnesium carbonate, silicified microcrystalline cellulose, powder cellulose and mannitol. Preferably, the filler is selected from microcrystalline cellulose, powder cellulose, silicified microcrystalline cellulose and calcium hydrogen phosphate, whereby microcrystalline cellulose is most preferably used.

Examples of disintegrants include sodium starch glycolate, croscarmellose sodium and crospovidone, whereby the most preferred disintegrant is crospovidone.

Examples of surfactants include polyoxyethylene stearate, sodium lauryl sulphate, sorbitan fatty acid esters (e.g. sorbitan monolaurate or sorbitan monopalmitate), polyoxyethylene sorbitan fatty acid esters (e.g. Polysorbate 80) and polyoxyethylene castor oil. However, it is preferred that the solid dispersion of the present invention does not contain a surfactant.

In a preferred embodiment of the present invention, the solid dispersion consists of ritonavir, the cationic polymer, a filler and a disintegrant. Preferably, the cationic polymer is an aminoalkyl methacrylate copolymer, e.g. butyl methacrylate/(2-dimethylaminoethyl)methacrylate/methyl methacrylate copolymer (1 : 2 : 1), the filler is microcrystalline cellulose, and the disintegrant is crospovidone.

The present invention also relates to a pharmaceutical composition for oral administration containing the solid dispersion of the present invention. The pharmaceutical composition may contain, besides the pharmaceutical excipients optionally contained in the solid dispersion, a filler, disintegrant and surfactant, which may be identical to those contained in the solid dispersion. The pharmaceutical composition may, additionally, contain a binder, a glidant and/or a lubricant.

Examples of binders include hydroxypropyl methylcellulose (HPMC), methylcellulose, hydroxypropyl cellulose (HPC), polyvinylpyrrolidone (povidone), polyvinyl alcohol, a vinylpyrrolidone/vinyl acetate copolymer (e.g. copovidone) and pregelatinized starch.

Examples of glidants include silicon dioxide (silica) and magnesium silicate.

Examples of lubricants include magnesium stearate, calcium stearate, zinc stearate, talc, sodium stearyl fumarate and glyceryl dibehenate.

The pharmaceutical composition of the present invention may be a tablet (including multiparticulate drug delivery systems) or a capsule that contains the solid dispersion of the present invention as particles (powder, granules or pellets), optionally in admixture with pharmaceutical excipients, and/or as minitablets.

The pharmaceutical composition of the present invention is preferably a tablet optionally coated with a film-coating. Commercially available film-coating systems containing polyvinyl alcohol as coating polymer, which are marketed under the tradename Opadry®, may be used.

The pharmaceutical composition may further contain a HIV protease inhibitor. If present, the HIV protease inhibitor is preferably not contained in the ritonavir-containing solid dispersion. The HIV protease inhibitor may be selected from darunavir, lopinavir, atazanavir, tipranavir and saquinavir. In a preferred embodiment, the pharmaceutical composition is an optionally film-coated multi-layer tablet containing ritonavir in a first layer and the HIV protease inhibitor in a second layer. For example, the multi-layer tablet may be an optionally film-coated bilayer tablet containing ritonavir in the first layer and darunavir in the second layer.

The pharmaceutical composition of the present invention is suitable, optionally in combination with other antiretroviral agents, for the treatment of HIV infection, e.g. HIV-1 infection.

The solid dispersion of the present invention may be prepared by melt-extrusion, spray-drying or spray-granulation, whereby spray-granulation is the preferred method. Hence, the solid dispersion of the present invention may be prepared by
i) dissolving ritonavir and the cationic polymer containing an amino group in a granulation liquid, and
ii) spraying the solution obtained in step (i) onto a pharmaceutical excipient to obtain the solid dispersion in form of granules.

Usually, the granulation liquid is an organic solvent, preferably an alcohol, such as ethanol. In a preferred embodiment of the present invention, the pharmaceutical excipient used in method step (ii) is selected from a filler and a disintegrant.

The pharmaceutical composition for oral administration according to the present invention may be prepared by
iii) mixing the solid dispersion of the present invention, which is preferably obtained by steps (i) and (ii) mentioned above, and a pharmaceutical excipient, and
iv) subjecting the mixture obtained in step (iii) to compression.

In a preferred embodiment of the present invention, the pharmaceutical composition contains a filler and disintegrant in the solid dispersion, i.e. as intragranular components, and a disintegrant, lubricant and glidant as extragranular components.

If a HIV protease inhibitor is additionally contained in the pharmaceutical composition, the HIV protease inhibitor is preferably not contained in the ritonavir-containing solid dispersion. This can be achieved by iii)
mixing the solid dispersion of the present invention, which is preferably obtained by steps (i) and (ii) mentioned above, a pharmaceutical excipient and the HIV protease inhibitor, and
iv) subjecting the mixture obtained in step (iii) to compression.

In this embodiment, the HIV protease inhibitor is contained in the pharmaceutical composition as extragranular component. Alternatively, the ritonavir-containing solid dispersion is contained in a first layer and the HIV protease inhibitor is contained in a second layer of an optionally film-coated multi-layer tablet. Preferably, the HIV protease inhibitor is darunavir.

The present invention also relates to a bilayer tablet containing ritonavir in a first layer and darunavir in a second layer. The optionally film-coated bilayer tablet may be prepared by
i) dissolving ritonavir and the cationic polymer containing an amino group in a granulation liquid,
ii) spraying the solution obtained in step (i) onto a pharmaceutical excipient to obtain a solid dispersion in the form of granules, and
iii) mixing the solid dispersion obtained in step (ii) and a pharmaceutical excipient,
v) subjecting a mixture containing darunavir and a pharmaceutical excipient to dry-granulation,
vi) mixing the granules obtained in step (v) and a pharmaceutical excipient,
vii) layering the mixture obtained in step (iii) onto the mixture obtained in step (vi),
viii) subjecting the two layers obtained in step (vii) to compression, and
ix) optionally subjecting the bilayer tablet obtained in step (viii) to film-coating.

Typically, the pharmaceutical excipient used in step (ii) is a filler and a disintegrant; the pharmaceutical excipient used in steps (iii) and (vi) is a disintegrant, a glidant and a lubricant; the pharmaceutical excipient used in step (v) is a filler and a lubricant.

The following examples are intended to further illustrate the present invention.

### Examples

| **INGREDIENT** | mg/bilayer tablet |
|---|---|
| ***Darunavir layer*** | |
| *Dry mix* | |
| Darunavir (amorphous) | 800 |
| Microcrystalline cellulose | 219.2 |
| Magnesium stearate | 5.6 |
| *Extragranular* | |
| Crospovidone | 78.4 |
| Colloidal silicon dioxide | 11.2 |
| Magnesium stearate | 5.6 |
| *Total weight of darunavir layer* | 1120 |

| ***Ritonavir layer*** | |
|---|---|
| *Intragranular* | |
| Ritonavir (form I) | 100 |
| Microcrystalline cellulose | 200 |
| Crospovidone | 49 |
| Eudragit® E 100 | 100 |
| EtOH | QS |

| *Extragranular* | |
|---|---|
| Crospovidone | 49 |
| Colloidal silicon dioxide | 10 |
| Sodium stearyl fumarate | 4.4 |
| COATING MATERIAL | 49 |

### Manufacturing process

### A. Darunavir layer

A-1: Sift all dry mix materials through appropriate sieve
A-2: Mix the sifted materials for appropriate time
A-3: Lubricate the mix with magnesium stearate
A-4: Compact the lubricated blend using roller compactor
A-5: Size the compacted flakes through appropriate sieve
A-6: Blend sifted granules for appropriate time
A-7: To the final sifted and blended granules of appropriate particle size distribution add crospovidone and colloidal silicon dioxide and mix for appropriate time
A-8: Lubricate the mixed product of previous step with magnesium stearate

### B. Ritonavir layer

B-1: Dissolve Eudragit® in ethanol
B-2: In the solution dissolve ritonavir and stir for appropriate time
B-3: Load microcrystalline cellulose in the fluid bed processor, along with crospovidone
B-4: Spray granulate with the solution of step B-2 to obtain a solid solution of ritonavir within a Eudragit® matrix
B-5: Mix the resulting granules with crospovidone and colloidal silicon dioxide for appropriate time
B-6: Lubricate with sodium stearyl fumarate

### C. Compression

Compress the two layers in a bilayer tablet

### D. Film-coating

Optionally, film-coat the bilayer tablet

## Claims

1. A solid dispersion comprising ritonavir, a cationic polymer containing an amino group, and optionally a pharmaceutical excipient.

2. The solid dispersion according to claim 1, wherein the weight ratio of the cationic polymer to ritonavir is 6 : 1 to 1 : 2, preferably 3 : 1 to 2 : 3, more preferred 3 : 2 to 1 : 1.

3. The solid dispersion according to claim 1 or 2, wherein the cationic polymer is selected from aminoalkyl methacrylate copolymer, polyvinylacetal diethylaminoacetate and chitosan.

4. The solid dispersion according to any one of the preceding claims, wherein the pharmaceutical excipient is selected from a filler, a disintegrant and a surfactant.

5. The solid dispersion according to claim 4, wherein the filler is selected from microcrystalline cellulose, powder cellulose, silicified microcrystalline cellulose and calcium hydrogen phosphate.

6. The solid dispersion according to claim 4, wherein the disintegrant is selected from sodium starch glycolate, croscarmellose sodium and crospovidone.

7. The solid dispersion according to claim 4, wherein the surfactant is selected from sorbitan monolaurate, sorbitan monopalmitate and polyoxyethylene castor oil.

8. The solid dispersion according to any one of the preceding claims, wherein the solid dispersion consists of ritonavir, a cationic polymer, a filler and a disintegrant.

9. The solid dispersion according to claim 8, wherein the cationic polymer is an aminoalkyl methacrylate copolymer, preferably butyl methacrylate/(2-dimethylaminoethyl)methacrylate/methyl methacrylate copolymer (1:2;1), wherein the filler is microcrystalline cellulose, and wherein the disintegrant is crospovidone.

10. A pharmaceutical composition for oral administration containing the solid dispersion as defined in any one of claims 1-9.

11. The pharmaceutical composition according to claim 10, wherein the composition further contains a HIV protease inhibitor.

12. The pharmaceutical composition according to claim 11, wherein the HIV protease inhibitor is not contained in the ritonavir-containing solid dispersion.

13. The pharmaceutical composition according to claim 11 or 12, wherein the HIV protease inhibitor is selected from darunavir, lopinavir, atazanavir, tipranavir and saquinavir.

14. The pharmaceutical composition according to any one of claims 11-13, wherein the composition is an optionally film-coated multi-layer tablet containing ritonavir in a first layer and the HIV protease inhibitor in a second layer.
